# EUROPÄISCHE PATENTSCHRIFT

(11) **EP 2 317 948 B1**
(45) Veröffentlichungstag und Bekanntmachung des Hinweises auf die Patenterteilung: **27.05.2015**
(21) Anmeldenummer: 09777224.8
(22) Anmeldetag: 16.07.2009
(51) Int. Cl.: A61B 17/72, A61B 17/00

(54) **IMPLANTATVORRICHTUNG ZUR GEWEBE- UND/ODER KNOCHENDISTRAKTION**
IMPLANT DEVICE FOR TISSUE AND/OR BONE DISTRACTION
DISPOSITIF IMPLANT POUR DISTRACTION TISSULAIRE ET/OU OSSEUSE

(30) Priorität: 30.07.2008 DE 102008035517
(43) Veröffentlichungstag der Anmeldung: 11.05.2011
(73) Patentinhaber: Storz-Irion, Regina, 78576 Emmingen-Liptingen (DE)
(72) Erfinder: STORZ-IRION, Regina, 78576 Emmingen-Liptingen (DE); GÜMPEL, Paul, 78351 Bodman-Ludwigshafen (DE); STRITTMATTER, Joachim, 78646 Konstanz (DE)
(74) Vertreter: Behrmann, Niels
(86) Internationale Anmeldenummer: PCT/EP2009/005161
(87) Internationale Veröffentlichungsnummer: WO 2010/012383

(56) Entgegenhaltungen:
- WO-A-98/47438
- DE-A1- 19 700 225
- DE-A1- 19 856 062
- US-A- 5 626 579

## Beschreibung

Die vorliegende Erfindung betrifft eine Implantatvorrichtung mit den Merkmalen des Hauptanspruchs. Eine derartige Vorrichtung ist aus dem Stand der Technik gemäß WO 9.8/47438 A bekannt, weiterer Stand der Technik ist etwa in der deutschen Patentschrift DE 198 10 639 bzw. der deutschen veröffentlichten Patentanmeldung DE 10 2007 036 359 der Anmelderin beschrieben.

Während traditionell und etwa seit Anfang des 20. Jahrhunderts zur Knochenverlängerung externe Fixateure eingesetzt werden, bei welchen Stangen von außen durch die Weichteile hindurch bis in die Knochen getrieben, über außerhalb liegende Ringelemente miteinander verbunden und täglich, typischerweise von Hand, justiert werden, um einen kontinuierlichen Zug auf die Knochenenden auszuüben und dadurch die Bildung von neuem Knochengewebe stimuliert wird, ist es seit einigen Jahren bekannt, Marknägel zur Knochenverlängerung zu benutzen, nämlich gattungsbildende Vorrichtungen derart, dass in einen Knochenmarkkanal operativ eine Implantatvorrichtung eingesetzt wird, welche zum Ausführen einer Streckbewegung durch externe Ansteuerung angeregt wird und so aus dem Knochen heraus das Knochenwachstum fördert.

Zu diesem Zweck offenbart beispielsweise die als DE 198 10 639 C2 eine Implantatvorrichtung, welche im Innenraum eine mittels einer rormgedächtnis-Legierung (FGL) realisierte Antriebsvorrichtung zum Bewirken des (typischerweise über mehrere zeitlich beabstandete Vorschubstufen erfolgenden)

Streckbetriebs ermöglicht. Durch Eintrag eines Hochfrequenz-Signals in die Vorrichtung erfolgt eine notwendige Energieversorgung der Antriebsvorrichtung, welche dann im gattungsgemäße Sinne von der Antriebsvorrichtur.g in einen gesteuerten, die Streckbewegung bewirkenden Vorschub umgesetzt wird. Eine vergleichbare Lösung offenbart die DE 10 2007 036 359 A1, welche zudem die Implantatvorrichtung in einen Steuer- und Überwachungskontext einbinden, so dass insbesondere der traditionelle manuelle Ansatz ersetzt werden kann durch eine automatisch gesteuerte, gleichwohl sorgfältig überwach- und dokumentierbare Therapie.

Allerdings setzen diese aus dem Stand der Technik bekannten Technologien, konstruktiv bedingt, einen Mindestdurchmesser des Markkanals voraus, um eine solche bekannte Vorrichtung einsetzen zu können; der realisierbare minimale Außendurchmesser eines solchen, bekannten Implantats macht daher eine Anwendung bei kleinen Röhrenknochen (z.B. bei menschlichen Armen) ebenso unmöglich, wie etwa einen Einsatz bei Menschen, welche generell einen Körperbau mit kleineren Knochen (z.B. Asiaten oder Kinder) besitzen. Damit können geschätzt etwa 70% der möglichen Patienten, bedingt durch zu geringe Knochendurchmesser, nicht von den beschriebenen Produkten aus dem Stand der Technik profitieren.

Aufgabe der vorliegenden Erfindung ist es, eine Vorrichtung nach dem Oberbegriff des Hauptanspruchs zu schaffen, welche im Hinblick auf anatomisch Bedingungen am Einsatzort, insbesondere einen realisierbaren minimalen Durchmesser des Implantats, kompakter gestaltet werden kann und damit auch geeignet ist, für Menschen mit kleinem. Knochendurchmesser (bzw. kleinen Innendurchmessern des Knochenmarkkanals) eingesetzt zu werden; insbesondere soll es Aufgabe sein, eine gattungsgemäße Implantatvorrichtung auch für eine Implantation bei Kindern geeignet zu machen.

Die Aufgabe wird durch die Vorrichtung mit den Merkmalen des Hauptanspruchs gelöst; vorteilhaft Weiterbildungen der Erfindung sind in den Unteransprüchen beschrieben.

In erfindungsgemäß vorteilhafter Weise werden dabei die die Streckbewegung bewirkenden Stellmittel realisiert durch eine Hydraulikstelleinheit, welche von einer außerhalb der Streckeinheit vorgesehenen Hytirauliksteuereinheit mit einem Hydraulikdruck versehen wird (wobei typischerweise die Hydrauliksteuereinheit intrakorporal, benachbart zu der Streckeinheit implantiert werden kann). Beide Aggregate sind über eine Druckleitung miteinander verbunden, so dass, aufgrund eines geringeren realisierbaren Durchmessers der typischerweise in Form eines Druckzylinders oder dergleichen realisierten Stellmittel in der Streckeinheit, diese im Außendurchmesser deutlich verringert werden kann, während die Aufgabe der Erzeugung des Hydraulikdrucks aus der Streckeinheit ausgelagert wird in die gehäuseexterne Hydrauliksteuereinheit.

Erfindungsgemäß wirken beide Einheiten dann zusammen, indem die Hydrauliksteuereinheit mit Hilfe eines ein thermisches und/oder magnetisches Formgedächtnislegierungs-Material aufweisenden Aggregats Hydraulikdruck erzeugt, welcher über die erfindungsgemäße Druckleitung bzw. das darin geführte Hydraulikfluid eingebracht wird in die Streckeinheit, welche mittels der erfindungsgemäßen Stellmittel und deren druckbedingter Längenveränderung zum Bewirken des therapeutisch wirksamen Streckvorgangs bewegt wird.

Erfindungsgemäß vorteilhaft ist dabei in der bevorzugten Weiterbildung das Hydraulikfluid ein biologisch und/oder medizinisch kompatibles Fluid, weiterbildungsgemäß eine physiologische Kochsalzlösung; auf diesem Wege wäre, im Fall einer unbeabsichtigten Öffnung des Hydraulik-Druckpfades, ein Austreten des Hydraulikfluids biologisch und medizinisch völlig unschädlich. Gleichzeitig sorgt das weiterbildungsgemäß vorgesehene, wasserhaltige Fluid für eine hinreichende Hydraulikwirkung.

Im Rahmen der Erfindung ist es zudem vorgesehen, magnetische, elektrische und/pder Wärmeenergie drahtlos und weiter bevorzugt von extrakorporal zuzuführen, wobei lediglich beispielhaft auf die in der zitierten DE 10 2007 036 359 A1 offenbarte Einkopplung von HF-Energie verwiesen wird. Diese technische Lehre gilt im Rahmen der Offenbarung der HF-Anbindung und HF-Energieeinkopplung im Rahmen der vorliegenden Anmeldung als Information. Entsprechendes gilt für die mit dieser Offenbarung zum Stand der Technik beschriebenen Einbettung in ein System, nämlich dadurch, dass auch über die HF-Anbindung eine Rückmeldung von geeigneten Betriebs- bzw. Störungssignalen nach extrakorporal erfolgen kann, so dass durch geeignete externe Ansteuerung eine kontinuierliche, schrittweise und zeitlich getaktete Knochenstreckung bei vollständiger Überwachung erfolgen kann; die weiterbildungsgemäß vorgesehenen Detektions- und Alarmsysteme ermöglichen diese systemische Einbindung.

Im Rahmen der Erfindung ist es weiterhin vorgesehen, die Streckeinheit mit einer Rastvorrichtung zu versehen, welche dafür sorgt, dass ein einmal bewirkter (typischerweise vorbestimmter) Vorschubhub bei einem Nachlassen des Hydraulikdrucks nicht zu einem Rückstellen führt, vielmehr ein kontinuierliches Bewegen -- im Takt der Rastung -- in lediglich einer Vorschubrichtung erfolgen kann.

Die weitere mechanische Realisierung, über die nachfolgende Beschreibung von Ausführungsbeispielen hinaus, ist offenbart in den zitierten Anmeldungen bzw. Patentschriften, wobei insbesondere die dort offenbarte mechanische Realisierung mittels zueinander verschiebbarer Hülsen als Gehäuse der Streckeinheit, die dort beschriebene Rastvorrichtung sowie die weitere endseitige Verankerung in den jeweiligen Endbereichen mit Knochen- bzw. Gewebebereichen als Information gelten soll.

Im Ergebnis ermöglicht es die vorliegende Erfindung, in überraschend einfacher und eleganter Weise einen erreichbaren minimalen Außendurchmesser einer insbesondere in einen Knochen zu implantierenden Vorrichtung so zu minimieren, dass damit die Nutzung von Knochenimplantaten zur Stimulierung der Knochengewebebildung völlig neuen Anwendungsfeldern und Patientengruppen zugänglich wird, insbesondere auch anatomisch begrenzten Implantationsräumen, wie etwa bei Kindern oder dergleichen. Damit ermöglicht die vorliegende Erfindung in vorteilhafter Weise auch den Einsatz als so genanntes mitwachsendes Implantatsystem, wie es etwa bei Kindern über einen längeren Therapiezeitraum nützlich und sinnvoll ist, auch eröffnet sich etwa der Bereich der Wirbelsäulenchirurgie mit der Möglichkeit der Korrektur von z. B. kindlichen Skoliosen.

Weitere Vorteile, Merkmale und Einzelheiten der Erfindung ergeben sich aus der nachfolgenden Beschreibung bevorzugter Ausführungsbeispiele sowie anhand der Zeichnungen - diese zeigen in
- Fig. 1: eine schematische Längsschnittansicht der Implantatvorrichtung gemäß einer ersten, bevorzugten Ausführungsform;
- Fig. 2: eine Detail-Schnittansicht der Hydrauliksteuereinheit gemäß Fig. 1;
- Fig. 3: eine Detail-Schnittansicht der Streckeinheit gemäß Fig. 1;
- Fig. 4: eine Seitenansicht der Vorrichtung gemäß Fig. 1 in einer axial um 90° gedrehten Ansicht, in einem im Knochen implantierten Zustand der Streckeinheit, und
- Fig. 5: ein schematisches Blockschaltbild eines Einsatzes der Implantatvorrichtung in einem Überwachungs- und Ansteuersystem.

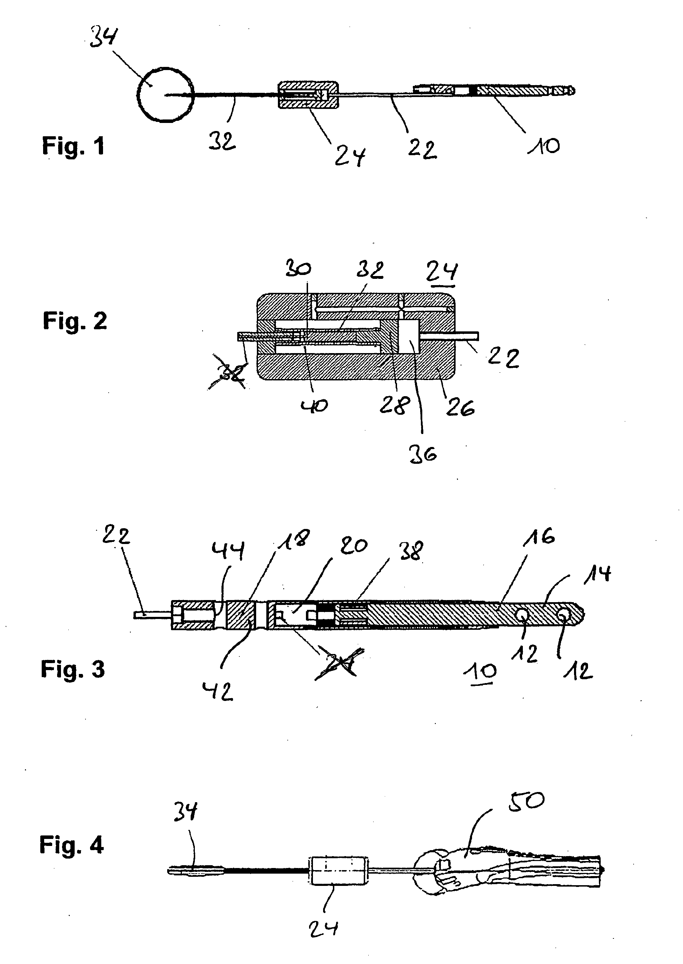

Eine in Fig. 1 rechts und in Fig. 3 im Detail gezeigte Streckeinheit 10 eines typischen Außendurchmessers von ca. 6 bis ca. 10mm ist zur Implantation in den Knochenmarkkanal eines geeignet zu streckenden (z. B. eine Fraktur aufweisenden) Knochens ausgebildet und weist zum Herstellen einer Verbindung mit dem Knochenmaterial einen zwei Schraubbohrungspaare 12 bzw. 44 aufweisenden Verankerungsabschnitt 14 bzw. 42 beidseits endseitig der Streckeinheit 10 auf (in Fig. 3 ist nur der rechtsseitige Verankerungsabschnitt 14 mit beiden Bohrungen 12, dagegen der linksseitige Verankerungsabschnitt 42, 44 schematisch gezeigt), wobei ein aus einem schaftartig-langgestreckt ausgebildeten Innenaggregat 16 und einem dieses teilweise hülsenartig umschließenden Außenschaft 18 gebildetes Gehäuse mittels einer Hydraulik-Druckkammer 20 so teleskopartig auseinanderbewegt werden kann, dass die jeweils endseitig am Innenschaft 16 bzw. Außenschaft 18 vorgesehenen Verankerungsabschnitte beim Auseinanderbewegen eine das beabsichtigte Knochenwachstum stimulierende Streckbewegung ausführen. Genauer gesagt wird das teleskopartige Auseinanderbewegen der Einheiten 16, 18 der Streckeinheit 10 bewirkt durch eine als physiologische Kochsalzlösung realisierte Hydraulikflüssigkeit, welche durch eine Hydraulikleitung 22 und einen Auslass in die Druckkammer 20 eintritt und das druckbedingte Auseinanderbewegen der Aggregate 16, 18 bedingt, wobei der Hydraulikdruck erzeugt wird durch eine anderenends der Druckleitung 22 vorgesehene und in Fig. 2 im Detail gezeigte Hydrauliksteuereinheit 24.

Die Hydraulikeinheit 24, im Ausführungsbeispiel der Fig. 2, ist realisiert als in einem Gehäuse 26 geführte Kolbeneinheit 28, welche als Antriebselement ein hohlzylindrisches, einen Endabschnitt des Kolbens 28 sowie ein Heizelement 30 umschließendes Hülsenelement 32 aus einem Formgedächtnismaterial realisiert ist.

Das Heizelement 30 wird dabei über eine mittels einer elektrischen Zuleitung angekoppelte Hochfrequenz-Empfängerspule (extrakorporal drahtlos angekoppelter) Hochfrequenzenergie versorgt, welche ein Aufheizen des Heizelementes auf eine Temperatur ermöglicht, bei welcher das thermisch gekoppelte Formgedächtnis-Legierungselement 32 eine Vorschubbewegung des Kolbens 28 und mithin einen Druck auf einen mit der Hydraulikflüssigkeit gefüllten, zur Hydraulikleitung 22 offenen Druckraum 36 ausübt.

Dieser durch HF-Einkopplung induzierte Druck (zur Wahl typischer Parameter wird verwiesen auf die Offenbarung gemäß DE 10 2007 036 359) wird mittels der Druckleitung 22 übertragen auf den Druckraum 20, welcher in der beschriebenen Weise eine Distraktion der Aggregate 16, 18 und mithin ein gesteuertes Auseinanderbewegen der jeweiligen endseitigen Verankerungsabschnitte 14 und 42 im Knochen bewirkt; randseitig am Innenaggregat 16 im Zusammenwirken mit einem Hülsenabschnitt des Außenaggregates 18 vorgesehene Rasteinheiten 38 (vergleiche hierzu etwa die DE 198 10 639 A1) verhindern, dass sich bei einem Druckabfall die Einheiten 16, 18 entgegen der Streckrichtung wieder teleskopartig aufeinander zu bewegen.

Ein in der Fig. 2 mit dem Bezugszeichen 40 versehener zylindrischer Bauraum, umgebend das Formgedächtnis-Legierungselement 32, bietet zusätzlich und weiterbildungsgemäß die Möglichkeit, eine Rückstellfeder einzusetzen und als Reservoir für Hydraulikflüssigkeit eingesetzt zu werden.

Die Fig. 4 verdeutlicht die Verankerung der Streckeinheit 10 in einem schematisch gezeigten Knochen 50; gut erkennbar ist, wie die Hydrauliksteuereinheit 24 sowie die HF-Antenne 34 im Körper, gleichwohl außerhalb der Streckeinheit (und, im gezeigten Ausführungsbeispiel auch außerhalb des Knochens) vorgesehen sind, wobei diese Einheiten auch im Knochen selbst vorgesehen sein könnten, ebenso wie die lediglich exemplarisch gerade gezeigten Leitungen 22 bzw. 32 geeignet flexibel, z. B. gekrümmt verlegt und entsprechend ausgestaltet sein können.

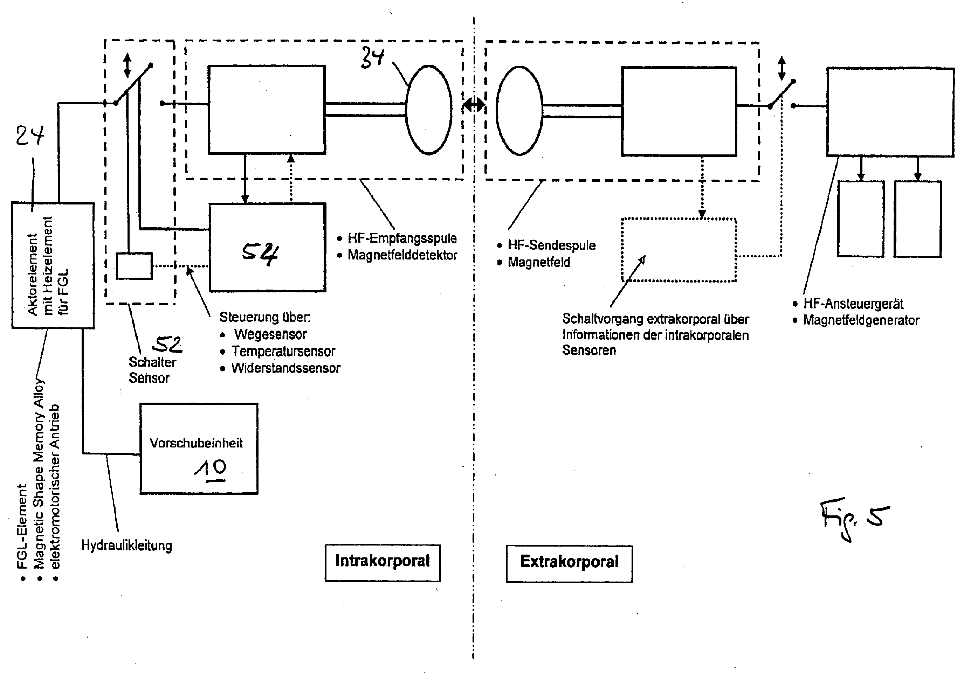

Die Fig. 5 zeigt im schematischen Blockschaltbild die Einbettung der erfindungsgemäßen Vorrichtung in ein Überwachungs- und Ansteuersystem, wobei die Einheiten der Fig. 1 bis 4 als Funktionsblöcke 10, 24 gezeigt sind und in der in Fig. 5 gezeigten (und, im Hinblick auf die An-steuer-,Schalt- und Überwachungsfunktionalität auch schon in der DE 10 2007 036 359 offenbarten Weise, welche insoweit als Information gelten soll) Weise beschrieben ist.

Konkret erfolgt mittels einer Schalter- bzw. Sensoreinheit 52 und einer zugehörigen Steuerungseinheit 54 eine gezielte Steuerüberwachung eines Distraktionsprozesses der Streckeinheit (Vorschubeinheit) 10 über eine Mehrzahl zeitlich aufeinander folgender und jeweils mit einem durch die Hydraulikeinheiten bestimmten Hub vorgenommenen Bewegung, wobei die Steuereinheit in Abhängigkeit von verschiedenen überwachten bzw. erfassten Parametern, etwa ein zurückgelegter Weg, eine erfasste FGL-Elementtemperatur oder dergleichen, die gewünschten Schritte auslöst. Extrakorporal und in der Art einer drahtlos vorgesehenen Versorgungs-, Steuerungs- und Überwachungseinheit findet sowohl die Einkopplung der HF-Energie (und/oder eines Magnetfeldes für das FGL-Element), als auch - durch Auswertung eines nach extrakorporal zurück übertragenen HF-Messsignals (bzw. Magnetfeldänderungssignals) - eine Auswertung, Aufzeichnung und Überwachung auf ordnungsgemäßen Betrieb bzw. Alarmzustand statt.

Die vorliegende Erfindung ist nicht auf die beschriebenen Ausführungsbeispiele beschränkt. Nicht nur bietet es sich an, die Erfindung - insbesondere im Hinblick auf einen erreichbaren Außendurchmesser der Streckeinheit - so auszubilden, dass damit ein Implantieren in Röhrenknochen, in Knochen von Kindern oder für andere anatomisch beengte Einsatzumgebungen möglich ist, auch ist ein Einsatz im Zusammenhang mit der Therapie von (kindlichen) Wirbelsäulenskoliosen möglich, ebenso wie die Verwendung der vorliegenden Implantatvorrichtung über einen längeren Zeitraum im implantierten Zustand als so genanntes mitwachsendes Implantatsystem, nämlich zur (therapeutischen) Begleitung von kindlichen Wachstumsprözessen.

## Patentansprüche

1. Implantatvorrichtung zur Gewebe- und/oder Knochendistraktion mit einer langgestreckten Streckeinheit (10), die zum Durchführen einer Streckbewegung zwischen zwei Verankerungsabschnitten (14, 42) für Gewebe und/oder Knochen ansteuerbar ist, wobei die Streckbewegung durch längenveränderliche Stellmittel (16, 10, 20) in der Streckeinheit bewirkt wird, wobei die Stellmittel eine Hydraulikstelleinheit aufweisen, die über eine Druckleitung (22) mit einer außerhalb der Streckeinheit vorgesehenen, intrakorporal der Streckeinheit benachbart vorsehbaren Hydrauliksteuereinheit (24) so zum Zusammenwirken verbunden ist, dass als Reaktion auf eine Druckbeaufschlagung eines in der Druckleitung geführten Hydraulikfluid durch die Hydrauliksteuereinheit die Hydraulikstelleinheit die Streckbewegung der Streckeinheit bewirkt,
wobei eine elektrische, elektromagnetische und/oder thermische Energieversorgung der Antriebsmittel in einem implantierten Zustand der Implantatvorrichtung mittels drahtlose Energieübertragungsmittel (34) erfolge, und wobei Detektormittel vorgesehen sind,
die senderseitig eine draht- und/oder berührungslose Feststellung eines Aktivierungs- und/oder Deaktivierungszustands oder Energieversorgung für die Antriebsmittel ermöglichen, und wobei die Streckeinheit eine Rastvorrichtung (38) aufweist, die eine schrittweise Streckbewegung entsprechend einem jeweiligen Rast- und/oder Zahnabstand der Rastvorrichtung bestimmt,
**dadurch gekennzeichnet, dass**
die Hydrauliksteuereinheit zur Druckbeaufschlagung mittels eines Formgedächtnis-Legierungselements aus einem thermischen und/oder magnetischen Formgedächtnis-Legierungsmaterial (32) realisierte Antriebsmittel aufweist,
wobei ein das Formgedächtnis-Legierungselement umgebender Bauraum (40) eine Rückstellfeder eingesetzt aufweist.

2. Vorrichtung nach Anspruch 1, **dadurch gekennzeichnet, dass** das Hydraulikfluid ein biologisch und/oder medizinisch verträgliches wasserhaltiges Fluid ist, insbesondere eine physiologische Kochsalzlösung.

3. Vorrichtung nach Anspruch 1, **dadurch gekennzeichnet, dass** die drahtlosen Energieübertragungsmittel eine Hochfrequenz-Sendeeinheit aufweisen, die in einem implantierten betriebszustand der Implantatvorrichtung zum Zusammenwirken mit einer Hochfrequenz-Empfangsspuleneinheit (34) der Implantatvorrichtung, insbesondere einer der Hydrauliksteuereinheit zugeordneten Hochfrequenz-Empfangsspuleneinheit, ausgebildet ist.

4. Vorrichtung nach einem der Ansprüche 1 bis 3, **dadurch gekennzeichnet, dass** den Detektormitteln eine integrierte Hinweis- und/oder Alarmeinheit zugeordnet ist, die so ausgebildet ist, dass als Reaktion auf ein Über- und/oder Unterschreiten vorbestimmter, bevorzugt zeitbezogener Schwellwerte zwischen zwei detektierten, aufeinanderfolgenden Änderungen der Aktivierung bzw. Deaktivierung der Energieversorgung ein Hinweis- und/oder Alarmsignal erzeugt wird.

5. Vorrichtung nach einem der Ansprüche 1 bis 4, **dadurch gekennzeichnet, dass** die Streckeinheit eine langgestreckte, mittels verschiebbarer Hülsen realisierte Gehäuseeinheit bestehend aus einem Außenrohr (18) und einem Innenaggregat (16) ) aufweist, welche zur Aufnahme der Stellmittel sowie zum bevorzugt axialen Herausführen der Druckleitung (22) ausgebildet ist.

6. Vorrichtung nach Anspruch 5, **dadurch gekennzeichnet, dass** die Hydraulikstelleinheit zwischen dem Außenrohr und dem schaftförmig ausgebildeten Innenaggregat so wirkt, dass als Reaktion auf die Druckbeaufschlagung ein Auseinanderbewegen von Außenrohr und Innenaggregat sowie ein entsprechendes Auseinanderbewegen zugehöriger Verankerungsabschnitte erfolgt.

## Claims

1. An implant device for tissue and/or bone distraction, with an elongate stretching unit (10), which can be controlled to perform a stretching movement between two anchoring portions (14, 42) for tissue and/or bone, wherein the stretching movement is effected by length-adjustable actuating means (16, 18, 20) in the stretching unit, wherein the actuating means have a hydraulic actuating unit connected via a pressure line (22) to a hydraulic control unit (24), which is provided outside the stretching unit and can be provided inside the body adjacent to the stretching unit, in order to interact therewith in such a way that, as a reaction to the hydraulic control unit applying a pressure to a hydraulic fluid guided in the pressure line, the hydraulic actuating unit effects the stretching movement of the stretching unit,
wherein an electric, electromagnetic and/or thermal energy supply of the drive means in an implanted state of the implant device takes place by means of wireless energy transmission means (34), and wherein detector means are provided, which on the transmitter side enable a wireless and/or contactless detection of an activation and/or deactivation state of the energy supply for the drive means,
and wherein the stretching unit has a detent device (38), which determines a gradual stretching movement in accordance with a respective detent- and/or tooth interval of the detent device,
**characterized in that**
the hydraulic control unit has drive means realized for the application of pressure by means of a shape memory alloy of a thermal and/or magnetic shape memory alloy material (32),
wherein a structural space (40) surrounding the shape memory alloy element has, in an inserted manner, a return spring.

2. The device according to Claim 1, **characterized in that** the hydraulic fluid is a biologically and/or medically compatible aqueous fluid, in particular a physiological saline solution.

3. The device according to Claim 1, **characterized in that** the wireless energy transmission means have a high frequency transmitting unit, which in an implanted operating state of the implant device is constructed for interaction with a high frequency receiver coil unit (34) of the implant device, in particular a high frequency receiver coil unit associated with the hydraulic control unit.

4. The device according to one of Claims 1 to 3, **characterized in that** an integrated indication and/or alarm unit is associated with the detector means, which indication and/or alarm unit is constructed such that an indication and/or alarm signal is generated as a reaction to an exceeding and/or falling below of predetermined, preferably time-related threshold values between two detected, successive changes to the activation or respectively deactivation of the energy supply.

5. The device according to one of Claims 1 to 4, **characterized in that** the stretching unit has an elongate housing unit, realized by means of displaceable sleeves, consisting of an outer tube (18) and an inner unit (16), which is constructed for receiving the actuating means and for the preferably axial guiding out of the pressure line (22).

6. The device according to Claim 5, **characterized in that** the hydraulic actuating unit between the outer tube and the inner unit, which is constructed in shaft form, acts such that as a reaction to the application of pressure a moving apart of outer tube and inner unit and a corresponding moving apart of associated anchoring sections takes place.

## Revendications

1. Dispositif d'implant pour la distraction tissulaire et/ou osseuse, comprenant une unité d'extension allongée (10) qui peut être commandée pour effectuer un mouvement d'extension entre deux sections d'ancrage (14, 42) pour des tissus et/ou des os, le mouvement d'extension étant réalisé par des moyens de commande de longueur variable (16, 18, 20) dans l'unité d'extension, les moyens de commande présentant une unité de réglage hydraulique qui est connectée par le biais d'une conduite de pression (22) à une unité de commande hydraulique (24) prévue à l'extérieur de l'unité d'extension, pouvant être prévue en position adjacente à l'unité d'extension à l'intérieur du corps de manière à coopérer de telle sorte qu'en réaction à une sollicitation par pression d'un fluide hydraulique conduit dans la conduite de pression par l'unité de commande hydraulique, l'unité de réglage hydraulique provoque le mouvement d'extension de l'unité d'extension,
une alimentation en énergie électrique, électromagnétique et/ou thermique des moyens d'entraînement dans un état implanté du dispositif d'implant s'effectuant au moyen de moyens de transfert d'énergie sans fil (34) et des moyens de détection étant prévus, lesquels permettent d'établir, du côté émetteur, sans fil et/ou sans contact, un état d'activation et/ou de désactivation de l'alimentation en énergie pour les moyens d'entraînement, et l'unité d'extension présentant un dispositif d'encliquetage (38) qui définit un mouvement d'extension pas à pas correspondant à une distance d'encliquetage et/ou une distance de dent respective du dispositif d'encliquetage,
**caractérisé en ce que**
l'unité de commande hydraulique présente, pour la sollicitation par pression au moyen d'un élément en alliage à mémoire de forme, des moyens d'entraînement réalisés à partir d'un matériau en alliage à mémoire de forme thermique et/ou magnétique (32),
un espace structurel (40) entourant l'élément en alliage à mémoire de forme présentant un ressort de rappel inséré.

2. Dispositif selon la revendication 1, **caractérisé en ce que** le fluide hydraulique est un fluide contenant de l'eau, biologiquement et/ou médicalement compatible, en particulier une solution physiologique de sel de cuisine.

3. Dispositif selon la revendication 1, **caractérisé en ce que** les moyens de transfert d'énergie sans fil présentent une unité émettrice haute fréquence qui est réalisée dans un état fonctionnel implanté du dispositif d'implant pour coopérer avec une unité de bobine réceptrice haute fréquence (34) du dispositif d'implant, en particulier une unité de bobine réceptrice haute fréquence associée à l'unité de commande hydraulique.

4. Dispositif selon l'une quelconque des revendications 1 à 3, **caractérisé en ce qu'**une unité d'indication et/ou d'alarme intégrée est associée aux moyens de détection, laquelle est réalisée de telle sorte qu'un signal d'indication et/ou d'alarme soit produit en réaction à un dépassement par le haut et/ou par le bas de valeurs_ seuil prédéterminées, de préférence rapportées au temps, entre deux variations successives détectées de l'activation ou de la désactivation de l'alimentation en énergie.

5. Dispositif selon l'une quelconque des revendications 1 à 4, **caractérisé en ce que** l'unité d'extension présente une unité de boîtier allongée, réalisée au moyen de douilles coulissantes, constituée d'un tube extérieur (18) et d'un groupe intérieur (16), qui est réalisée pour recevoir les moyens de commande et pour faire ressortir de préférence axialement la conduite de pression (22).

6. Dispositif selon la revendication 5, **caractérisé en ce que** l'unité de réglage hydraulique agit entre le tube extérieur et le groupe intérieur réalisé en forme de tige de telle sorte qu'un écartement du tube extérieur et du groupe intérieur ait lieu en réaction à la sollicitation par pression, ainsi qu'un écartement correspondant de portions d'ancrage associées.
